(19) Europäisches Patentamt
European Patent Office
Office européen des brevets

(11) Publication number: **0 075 542**
**B1**

(12) **EUROPEAN PATENT SPECIFICATION**

(45) Date of publication of patent specification: **16.04.86**

(51) Int. Cl.⁴: **A 61 M 16/00**

(21) Application number: **82830230.7**

(22) Date of filing: **09.09.82**

(54) **Apparatus for artificial pulmonary ventilation during anaesthesia and resuscitation.**

(30) Priority: **22.09.81 IT 2407481**

(43) Date of publication of application:
**30.03.83 Bulletin 83/13**

(45) Publication of the grant of the patent:
**16.04.86 Bulletin 86/16**

(84) Designated Contracting States:
**AT BE CH DE FR GB LI LU NL SE**

(56) References cited:
**FR-A- 477 277**
**FR-A-2 068 581**
**FR-A-2 221 152**
**FR-A-2 368 942**
**US-A-2 914 064**
**US-A-4 239 039**
**US-A-4 262 689**

(73) Proprietor: **SOXIL S.p.A. GRUPPO PIERREL**
**Via Antonelli 3**
**I-20139 Milano (IT)**

(72) Inventor: **Rodari, Gianpiero S.**
**Viale Europa 1**
**Bussero (Milan) (IT)**

(74) Representative: **De Nova, Roberto et al**
**c/o Jacobacci-Casetta & Perani S.n.c. Via**
**Visconti di Modrone 7**
**I-20122 Milano (IT)**

Courier Press, Leamington Spa, England.

## Description

The present invention relates to apparatus for artificial pulmonary ventilation during anaesthesia and resuscitation.

As is known, during anaesthesia and resuscitation, it is necessary to carry out artificial pulmonary ventilation on a patient when natural respiration is inactive or in any way insufficient. This ventilation essentially involves supplying the patient's lungs with air, which may be humidified, enriched with oxygen or mixed with anaesthetic gases, as necessary.

At present, there are basically two different techniques available for carrying out this ventilation.

According to a first method, excess external pressure is established in a certain rhythm which is approximately equal to the natural respiratory rhythm or is synchronised therewith. For the entire time during which this excess pressure is maintained there is a real transport of air into the lungs, this commonly being called "mass transport" or "bulk transport"; on cessation of the excess pressure there is a similar transport in the opposite direction. The control of the ventilation may be effected by controlling the pressure or the volume of the air supplied.

This method has several disadvantages: for example, the excess pressure disturbs the cardiac output, hindering the circulation of blood, compresses the blood in the pulmonary alveoli, and may cause pulmonary lesions (barotrauma) in patients with fibrous pulmonary tissue. Moreover, it does not allow surgical operations to be carried out on the trachea because of the rhythmic movement caused therein.

According to a second method, the transport of air, and particularly the transport of oxygen towards the pulmonary alveoli and the removal of carbon dioxide from the alveoli, is effected by diffusion ("diffusion transport"). In order to ensure a sufficient rate of diffusion, however, it is necessary to establish an oscillation of the air at a suitable frequency. In apparatus at present available for carrying out this method, the air is oscillated by periodically stopping a flow of pressurized air in a supply line by means of a solenoid valve. The flow of air is thus interrupted, resulting in the creation of a succession of wave fronts, and hence an oscillation proper. Although advantageous in that it is free from the disadvantages mentioned above, this ventilation apparatus still has only limited use.

Above all, it requires very frequent replacement of the solenoid valve which, because of the frequency of operation, is subject to very heavy use and soon reaches the end of its working life. A second disadvantage lies in the fact that the frequency of oscillation which may be imparted to the air is relatively low because of the limits on the frequency with which the solenoid valve may be energised and de-energised.

In order to overcome these disadvantages US—A—2.914.064 discloses an apparatus for artificial pulmonary ventilation during anaesthesia and resuscitation, including an on-off valve in a line for supplying air to the lungs, the valve having a valve body, a rotor seat in the valve body, respective air inlet and outlet ports in the valve body which open into the seat, a rotor sealingly rotatable in the seat under the action of drive means, and at least one duct in the rotor for periodically interconnecting the ports in accordance with the rotation of the rotor.

Although advantageous in that it is free from the disadvantages mentioned above, this apparatus does not permit to vary the duration of the connection according to a desired periodic distribution of the duration of the connection and the duration of the interruption.

The problem behind the present invention is that of devising apparatus of the type specified above, which has structural and functional characteristics such as to overcome the disadvantages mentioned above with reference to the prior art.

This problem is solved by apparatus of the type specified, which is characterised in that the valve is divided into two parts which are angularly displaceable relative to each other, each part comprising one of the two ports.

Further characteristics and advantages of the apparatus according to the present invention will emerge from the following description of a preferred embodiment, given by way of non-limiting example with reference to the appended drawings, in which:

Figure 1 is a schematic view of apparatus according to the invention;

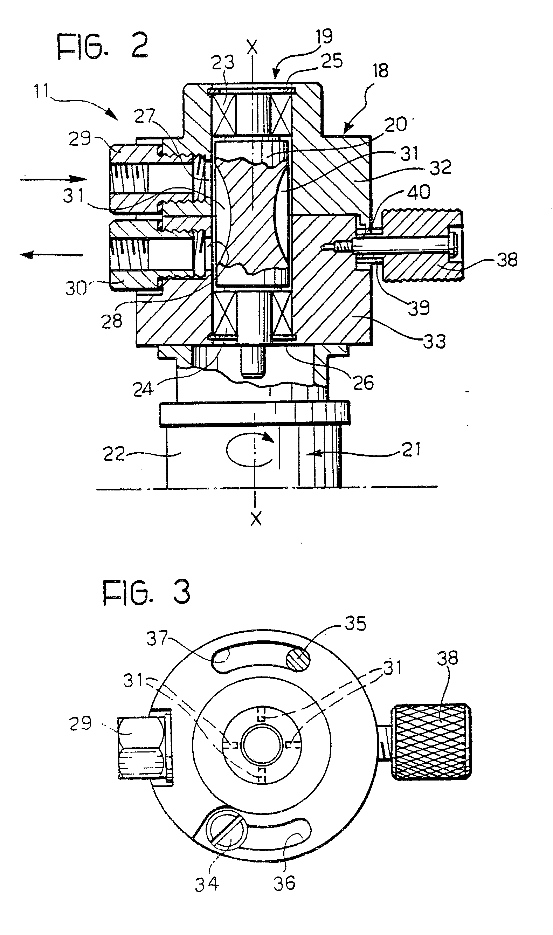

Figure 2 is a partially sectioned elevational view of an on-off valve used in the apparatus of Figure 1;

Figure 3 is a plan view of the valve of Figure 2;

Figure 4 is a side view of the valve of Figure 2;

Figure 5 shows a variant of the valve of Figure 2.

With reference to the appended drawings, apparatus for artificial pulmonary ventilation during anaesthesia and resuscitation is generally indicated 1.

As will be clarified in the description below, this apparatus is based on the diffusion method and, more particularly, is of the high frequency jet ventilation (HFJV) type.

The apparatus 1 includes a Venturi tube 2 with an opening 3 which communicates with the environment and an opening 4 for connection to the thoracic cavity of a patient through an endotracheal tube 5.

Within the Venturi tube 2 is located coaxially the end 6 of an air supply line 7 with a mouth 8 which opens into the constriction of the Venturi tube 2 to act as a jet nozzle.

The opposite end 9 of the line 7 is connected to a mixer 10 in which the air is enriched with oxygen or mixed with anaesthetic gases, as required.

The line 7, through which air flows in the direction of arrow F, includes an on-off valve 11 which will be described in detail below.

A tube 12 branches from the line 7 at a point 13 upstream of the on-off valve 11 and opens into the Venturi tube 2 through an aperture 14 formed in the wall of the tube substantially in the constriction.

A pressure reducing valve 15, its pressure gauge 16, and a humidifier 17 are located in succession along the tube 12.

The on-off valve 11 includes a valve body 18 in which a cylindrical rotor seat 19 with an axis X—X is formed. Within the seat 19 is housed a rotor 20 which is sealingly rotatable in the seat 19 about the axis X—X under the action of drive means, generally indicated 21, including a variable-speed electric motor 22.

In particular, the rotor 20 is supported by bearings 23, 24 and is retained axially by respective retaining rings 25, 26 housed in the valve body 18.

Two inlet and outlet ports 27, 28 provided with respective connectors 29, 30 are formed in the valve body 18 and have rectangular openings into the rotor seat 19 aligned substantially in the direction X—X.

The rotor 20 is provided with four ducts, each indicated 31, which are formed at regular intervals around the periphery of the rotor and extend in the direction X—X for such length as to take in the two ports 27, 28.

The body 18 is formed in two parts 32, 33 which are juxtaposed in a plane perpendicular to the axis X—X. The parts 32, 33 carry the respective ports 27, 28 and are angularly displaceable to move these ports out of alignment with each other.

The two parts 32, 33 are maintained in juxtaposition by two screws 34, 35 which are captive in the part 33 and are engaged with two slots 36, 37 formed in the part 32.

A knob 38 is fixed rotatably on the part 33 and is provided with a pinion 39 which is engaged with toothing 40 formed on the part 32 to cause the relative angular displacement of the parts 32, 33.

The operation of the apparatus 1 is described below.

From the mixer 10, the air is directed through the line 7 towards the Venturi tube 2. When the flow of air passes through the on-off valve 11 the motor 22 of which is rotated at a predetermined angular velocity, it is periodically interrupted and hence, so to speak, broken. In fact, the inlet port 27 and the outlet port 28 are periodically interconnected by the ducts 31, when the latter are brought into positions facing the ports 27, 28 by the rotation of the rotor.

Clearly, in addition to the angular velocity of the duration of this connection also depends on the transverse dimensions of the ports 27, 28 and the ducts 31 together with the relative positioning of the two ports 27, 28 which are more or less out of alignment with each other.

It is possible to vary the non-alignment of the two ports by means of the knob 38 and thus vary the duration of the connection.

The duration of the connection is chosen according to a desired periodic distribution of the duration of the connection and of the interruption. Normally, it is arranged that the duration of the connection is less than that of the interruption, so as to favour the diffusion of carbon dioxide leaving the lungs relative to the diffusion of the entering oxygen. For example, it is arranged that, in the entire period, about 40% thereof comprises connection and about 60% interruption.

The flow of air which is interrupted in this way reaches the Venturi tube 2 where it mixes with the flow of suitably humidified air coming from the tube 12. The mixture thus obtained is then placed at the disposal of the patient's lungs by means of the endotracheal tube 5. The oscillation of the mixture in the entire volume of the lungs is maintained by the interrupted flow from the mouth 8 of the line 7, these oscillations favouring the gaseous diffusion of the gases within the volume of the lungs, and thus favouring the transport of oxygen into the lungs as well as the removal of carbon dioxide.

The apparatus according to the invention has the main advantages of high reliability and a long working life which are achieved essentially by virtue of the particular rotary operation of the on-of valve 11. In addition to this, with the apparatus according to the invention, the frequency of oscillation may be brought to very high values because of the ease with which the on-off valve 11 may be constructed with a very large number of ducts 31.

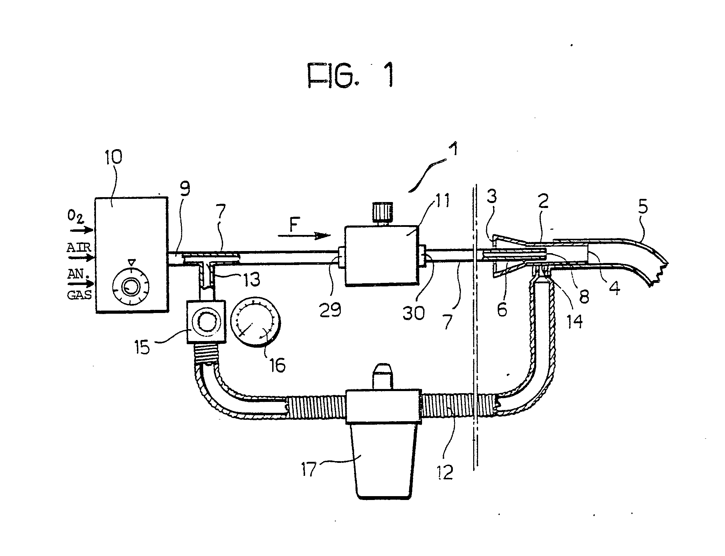

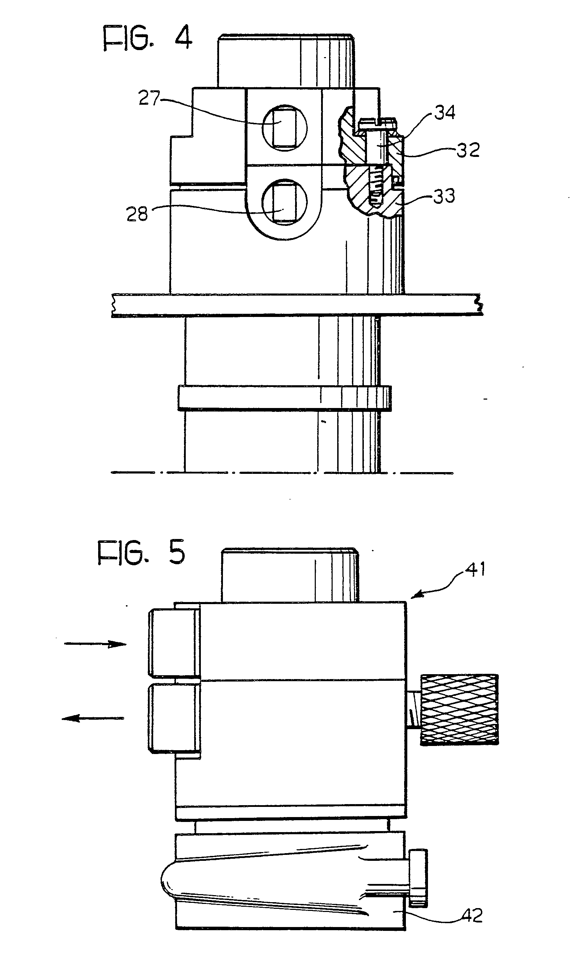

Figure 5 shows an on-off valve 40 which is a variant of the on-off valve 11 of Figure 1. In Figures 1 and 5, similar parts are indicated by the same reference numerals. In the valve 41, the drive means for rotating the rotor 20 comprise an air turbine 42 supplied with compressed air in a conventional manner.

By virtue of this variation, the ventilating apparatus according to the invention has the advantage, which is appreciable in some situations, of complete independence from the mains supply and hence has greater versatility in use.

**Claims**

1. Apparatus (1) for artificial pulmonary ventilation during anaesthesia and resuscitation including an on-off valve (11) in a line (7) for supplying air to the lungs, the valve (11) having a valve body (18), a rotor seat (19) in the valve body (18), respective air inlet and outlet ports (27, 28) in the valve body (18) which open into the seat (19), a rotor (20) sealingly rotatable in the seat (19) under the action of drive means (21), and at least one duct (31) in the rotor (20) for periodically interconnecting the ports (27, 28) in accordance with the rotation of the rotor (20), characterised in that the valve body (18) is divided into two parts (32, 33) which are angularly displaceable relative to each other, each part (32, 33) comprising one of the two ports (27, 28).

2. Apparatus according to Claim 1, characterised in that said drive means (21) comprises a variable speed electric motor (22).

3. Apparatus according to Claim 1 characterised in that the drive means (21) comprises a turbine (42).

## Patentansprüche

1. Vörrichtung (1) zur Künstlichen Beatmung während einer Anästhesie oder einer Wiederbelebung, enthältend in einer Leitung (7) Zuführen von Luft zu den Lungen ein Ventil (11), das einen Ventilkörper (18), eine Rotoraufnahme (19) in dem Ventilkörper (18), jeweils zu der Aufnahme (19) öffnende Lufteinlaß- und Luftauslaßöffnungen (27, 28) in dem Ventilkörper (18), einen abgedichtet unter der Wirkung von Antriebsmitteln (21) in der Aufnahme (19) drehbaren Rotor (20) und zum periodischen Verbinden der Offnungen (27, 28) entsprechend der Rötation des Rotors (20) wenigstens eine Verbindung (31) in dem Rotor (20) hat, dadurch gekennzeichnet, daß der Ventilkörper (18) in zwei Teile (32, 33) unterteilt ist, die winklig relativ zueinander bewegbar sind, wobei jeder der beiden Teile (32, 33) eine der Offnungen (27, 28) enthält.

2. Vorrichtung nach Anspruch 1, dadurch gekennzeichnet, daß die Antriebsmittel (21) einen drehzahlveränderlichen Elektromotor (22) enthalten.

3. Vorrichtung nach Anspruch 1, dadurch gekennzeichnet, daß die Antriebsmittel (21) eine Turbine (42) enthalten.

## Revendications

1. Appareil pour la ventilation pulmonaire artificielle durant l'anesthésie et la réanimation, comportant une vanne (11) sur un tuyau (7) d'alimentation d'air aux poumons, la vanne (11) présentant un corps (18), un logement (19) dans ce corps (18) pour un rotor, orifices (27, 28) dans ce corps (18) respectivement d'entrée et de sortie de l'air, ouverts dans ce logement (19), un rotor (20) tournant à étanche dans ce logement (19) sous action de moyens de commande, et au moins un conduit (31) dans ce rotor (20) pour mettre périodiquement en communication entre eux ces orifices (27, 28) suivant la rotation du rotor (19), caractérisé par le fait que ce corps (18) est partagé en deux parties (32, 33) lesquelles sont déplaceables angulairement l'une par rapport à l'autre, chaque partie (32, 33) comportant un des deux orifices (27, 28).

2. Appareil selon la revendications 1, caractérisé par le fait que ces moyens de commande (21) comportent un moteur électrique à vitesse variable (22).

3. Appareil selon la revendication 1, caractérisé par le fait que ces moyens de commande (21) comportent une turbine (42).

0 075 542

FIG. 1

FIG. 2

FIG. 3

FIG. 4

FIG. 5